# EUROPEAN PATENT SPECIFICATION

(11) **EP 2 455 127 B1**
(45) Date of publication and mention of the grant of the patent: **24.12.2014**
(21) Application number: 10799348.7
(22) Date of filing: 13.07.2010
(51) Int. Cl.: A61M 5/50, A61M 5/178, A61M 5/32

(54) **SAFE DISPOSABLE INJECTOR WITH CHANGEABLE AND AUTOMATICALLY RETRACTABLE NEEDLE**
SICHERER EINWEG-INJEKTOR MIT AUSTAUSCHBARER UND AUTOMATISCH EINZIEHBARER NADEL
INJECTEUR JETABLE SÛR ÉQUIPÉ D'UNE AIGUILLE INTERCHANGEABLE ET SE RÉTRACTANT AUTOMATIQUEMENT

(30) Priority: 15.07.2009 CN 200910164711
(43) Date of publication of application: 23.05.2012
(73) Proprietor: Wuxi Yushou Medical Appliances Co., Ltd., Dongbeitang, Xishan District Wuxi Jiangsu 214191 (CN)
(72) Inventor: JIANG, Wenjun, Jiangsu 212300 (CN)
(74) Representative: Franks & Co Limited
(86) International application number: PCT/CN2010/001046
(87) International publication number: WO 2011/006350

(56) References cited:
- EP-A1- 1 977 777
- EP-A2- 0 747 075
- WO-A1-2006/096901
- CN-A- 101 094 704
- CN-A- 101 601 882
- CN-U- 201 481 922
- CN-U- 201 481 935
- CN-Y- 2 880 122
- CN-Y- 201 098 441
- US-A1- 2002 173 753
- US-A1- 2003 023 205
- US-A1- 2004 199 113
- US-A1- 2009 124 971

## Description

### Background of the Present Invention

### Field of Invention

The present invention relates to an injector, and more particular to a safe disposable injector, wherein the injecting needle is changeable and retractable after use.

### Description of Related Arts

It is common practice for a disposable syringe to administer injections and vaccinations since the disposable syringe is a one-time-use device for preventing infectious diseases and nosocomial cross-infection. Such disposable syringe, such as syringe with auto-destroy needle, syringe with manual retractable needle, or syringe with automatic retractable needle, are generally of the disposable nature and are normally discarded after a single use. However, the disposable syringe has complicated structural configuration and lacks of necessary operational function. For example, when preparing dry powder injection, the syringe is used for injecting solvent into a sealed bottle in order to dissolve the dry powder in liquid form. In particular, the needle must be penetrated through the bottle stopper in order to inject the solvent into the bottle. In other words, the needle will be bent or deformed during the penetration. In addition, the tip of the needle will be blunt and the smoothness of the needle body will be substantially reduced after the penetration. Therefore, the syringe must be discarded after the preparation of the dry powder injection since the needle cannot be re-used to administer injection for human body. In fact, the syringe is prohibited for being re-used after preparation of the dry powder injection in many countries. Therefore, the physician must use one disposable syringe to prepare the dry powder injection and another one to administer injection. Accordingly, there is no syringe with changeable needle in the market, especially for the retractable needle.

EPA1977777 discloses an improved barrel type plunger for use with a needle-retractable safety syringe.

### Summary of the Present Invention

The invention is advantageous in that it provides a safety disposable injector, which solves the existing problems of the syringe, wherein the needle is changeable and automatically retractable after use. The present invention is convenient to use and reliable to operate. After injection, the injector needle can be retracted by an automatic retraction mechanism to avoid an injury by accidental needle sticks as serious health hazard and danger of exposure to fatal blood-transmitted viruses.

Additional advantages and features of the invention will become apparent from the description which follows, and may be realized by means of the instrumentalities and combinations in the appended claims.

According to the present invention, the foregoing and other objects and advantages are attained by a safety disposable injector comprising a barrel and a plunger slidably coupling within the barrel in a reciprocal manner.

The present invention comprises a safety disposable injector as defined in claim 1.

The injecting needle protection sleeve further has a handling wing, a rhombus cross section (non-circular cross section), or knurled outer surface for enabling the operator to hold and turn the injection needle unit to attach to the barrel.

Accordingly, the present invention provides the advantages as follows:
(1) According to the need of the injection, the operator is able to selectively interchange between the medicament-dispensing needle unit and the injecting needle unit to couple with the barrel. After the preparation of the liquid by the medicament-dispensing needle unit, the injecting needle unit to couple with the barrel to administer injections. Therefore, the sharpness of the injecting needle and the smoothness of the injecting needle will always be maintained at the best condition.
(2) The operation of the present invention is the same as the convention injection operation that the conventional operation habit remains basically unchanged in the present invention. The present invention is convenient to use and reliable to operate. After injection, the injector needle can be retracted by an automatic retraction mechanism to avoid the accidental hurt to medical staff and patients by a polluted needle tip.
(3) The present invention has a simple structural configuration and is suitable for automatic production on the assembly line, so that the present invention is capable of maintaining a high quality and reducing the manufacturing cost.
(4) As a result, the present invention will substantially reduce the unnecessary waste of material by interchanging the medicament-dispensing needle unit and the injecting needle unit, such that present invention is considered as an environmental friendly product.

Still further objects and advantages will become apparent from a consideration of the ensuing description and drawings.

These and other objectives, features, and advantages of the present invention will become apparent from the following detailed description, the accompanying drawings, and the appended claims.

### Brief Description of the Drawings

Fig. 1 is a sectional view of a safety disposable injector with medicament-dispensing needle unit according to a preferred embodiment of the present invention, illustrating the wing-type medicament-dispensing needle unit.
Fig. 2 is a sectional view of the safety disposable injector with medicament-dispensing needle unit according to the above preferred embodiment of the present invention, illustrating the rhombus-type medicament-dispensing needle unit.
Fig. 3 is a sectional view of the safety disposable injector with injection needle unit according to the above preferred embodiment of the present invention.
Fig. 4 is a sectional view of the safety disposable injector with injection needle unit according to the above preferred embodiment of the present invention, illustrating the injection needled unit being retracted.
Fig. 5 is a sectional view of the injection needle unit of the safety disposable injector according to the above preferred embodiment of the present invention.
Fig. 6 is a sectional view of the medicament-dispensing needle unit of the safety disposable injector according to the above preferred embodiment of the present invention.
Fig. 7 is a sectional view of the injection needle unit of the safety disposable injector according to the above preferred embodiment of the present invention, illustrating the rhombus-type injection needle unit.
Fig. 8 is a sectional view of the medicament-dispensing needle unit of the safety disposable injector according to the above preferred embodiment of the present invention, illustrating the rhombus-type medicament-dispensing needle unit.
Fig. 9 illustrates a sealing structure of the barrel of the safety disposable injector with injection needle according to the above preferred embodiment of the present invention.
Fig. 10 illustrates an automatic retraction mechanism of the safety disposable injector with injection needle according to the above preferred embodiment of the present invention.

### Detailed Description of the Preferred Embodiment

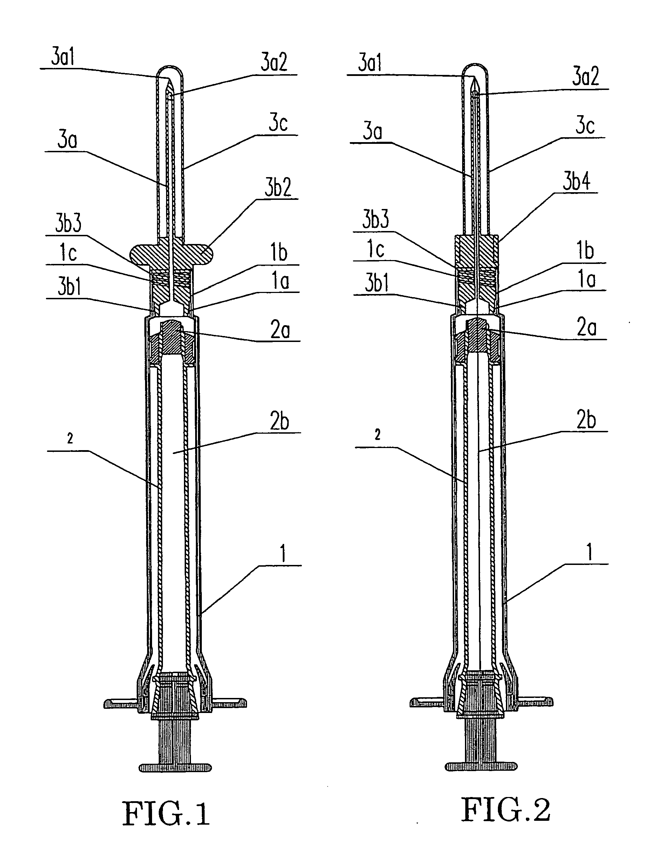

Referring to Figs. 1 to 6 of the drawings, a safety disposable injector, such as a disposable syringe, according to a preferred embodiment of the present invention is illustrated, wherein the safety disposable injector comprises a barrel 1 having a chamber, and a plunger 2 slidably coupling within the chamber of the barrel 1 in a reciprocal manner. The safety disposable injector further comprises a medicament-dispensing needle unit 3 and an injecting needle unit 4 selectively coupling with the barrel 1. According to the preferred embodiment, the medicament-dispensing needle unit 3 comprises a medicament-dispensing needle body 3a and a medicament-dispensing needle base 3b, wherein the medicament-dispensing needle body 3a is fixed and upwardly extended from the medicament-dispensing needle base 3b. Alternatively, the medicament-dispensing needle body 3a and the medicament-dispensing needle base 3b of the medicament-dispensing needle unit 3 can be formed in a one piece integrated structure by mold injection. The injecting needle unit 4 comprises an injecting needle body 4a and a built-in automatic retraction mechanism which comprises an injecting needle base 4b, a base sleeve 4c, a sealing element 4f which is preferably a sealing O-ring, a compression spring 4d, and a spring seat 4e. The injecting needle body 4a is upwardly and coaxially extended from the injecting needle base 4b that a bottom portion of the injecting needle body 4a is held by the injecting needle base 4b. The base sleeve 4c, which is a tubular member, comprises a retention protrusion 4c1 protruded from an inner wall of the base sleeve 4c at a bottom end thereof, wherein the sealing element 4f is supported within the base sleeve 4c by the retention protrusion 4c1 so as to retain the sealing element 4f at the bottom end of the base sleeve 4c. The injecting needle base 4b is held within an interior cavity of the base sleeve 4c to define a gap therebetween. The spring seat 4e is coupled at a top end of the base sleeve 4c, wherein the spring seat 4e can be fixed to the base sleeve 4c by clamp-engagement, rotary-engagement, integrated structure or the like. The injecting needle body 4a is extended from the interior cavity of the base sleeve 4c to an exterior of the injecting needle base 4b through the spring seat 4e. The compression spring 4d is coaxially coupled with the injecting needle base 4b and is retained between the spring seat 4e and a bottom end of the injecting needle base 4b. Accordingly, the top end of the compression spring 4d is biased against the spring seat 4a while the bottom end of the compression spring 4d is biased against the bottom end of the injecting needle base 4b such that the compression spring 4d will apply a compression spring force against the injecting needle base 4b. In addition, the sealing element 4f is sealed and retained between the bottom end of the base sleeve 4c at the retention protrusion 4c1 thereof and the bottom end of the injecting needle base 4b to provide a sealing effect therebetween and to apply a frictional holding force against the injecting needle base 4b so as to withstand the compression force of the compression spring 4d. In other words, the sealing element 4f is provided at the gap between the injecting needle base 4b and the base sleeve 4c. Accordingly, the frictional holding force of the sealing element 4f equals to or greater than the compression force of the compression spring 4d, such that the injection needle base 4b will be retained in position and the compression spring 4d is retained at a compressed state. In other words, the injection needle base 4b is held within the base sleeve 4c via the sealing element 4f. The barrel 1 has an inner threaded portion 1c. Correspondingly, the medicament-dispensing needle unit 3 further has an outer threaded portion 3b3 while the injecting needle unit 4 further has an outer threaded portion 4c5. According to the operation of the present invention, the medicament-dispensing needle unit 3 and the injecting needle unit 4 can be selectively coupled with the barrel 1 preferably by rotatably engaging the outer threaded portion 3b3 of the medicament-dispensing needle unit 3 to the inner threaded portion 1c of the barrel 1 or by rotatably engaging the outer threaded portion 4c5 of the injecting needle unit 4 to the inner threaded portion 1c of the barrel 1. It is appreciated that other engagements such as plug-in engagement or clip-on engagement can be used for selectively and detachably coupling one of the medicament-dispensing needle unit 3 and the injecting needle unit 4 with the barrel 1. It is worth mentioning that the top opening end portion of the barrel 1 has an inner diameter gradually reducing toward the bottom end thereof to form an inner conical opening 1a at an inner surface of the barrel 1. Correspondingly, each of the medicament-dispensing needle unit 3 and the injecting needle unit 4 further has an outer conical platform 3b1, 4c3 formed at an outer circumferential surface of the bottom end of each of the medicament-dispensing needle unit 3 and the injecting needle unit 4. It is worth mentioning that each of the medicament-dispensing needle unit 3 and the injecting needle unit 4 can have a circular platform instead of the outer conical platform 3b1, 4c3. When the medicament-dispensing needle unit 3 or the injecting needle unit 4 is rotatably engaged with the barrel 1, the outer conical platform 3b1, 4c3 of the medicament-dispensing needle unit 3 or the injecting needle unit 4 will bias against the inner conical opening 1a of the barrel 1 so as to create a sealing effect between the outer conical platform 3b1, 4c3 of the medicament-dispensing needle unit 3/the injecting needle unit 4 and the inner conical opening 1a of the barrel 1. If the taper of the inner conical opening 1a of the barrel 1 is different from the taper of the outer conical platform 3b1, 4c3 of the medicament-dispensing needle unit 3 or the injecting needle unit 4, i.e. the taper of the inner conical opening 1a of the barrel 1 is larger than the taper of the outer conical platform 3b1, 4c3 of the medicament-dispensing needle unit 3 or the injecting needle unit 4, the sealing effect between the two surfaces will be achieved. For the best mode, when the taper of the inner conical opening 1a of the barrel 1 matches with the taper of the outer conical platform 3b1, 4c3 of the medicament-dispensing needle unit 3 or the injecting needle unit 4, the sealing effect between the surfaces will be maximized by increasing the contacting surface area. It is appreciated that an O-shaped sealing ring or other sealer element can be used for sealing between the two surfaces to further enhance the sealing effect therebetween. In order to operate the present invention, the medicament-dispensing needle unit 3 is firstly coupled with the barrel 1 such that by slidably pulling the plunger 2, the solvent is sucked into the barrel 1 via the medicament-dispensing needle unit 3. Then, by inserting the medicament-dispensing needle unit 3 into a bottle containing the dry powder medication, the solvent is injected into a bottle containing the dry powder medication via the slidably pushing the plunger 2, so as to dissolve the dry powder medication to form a liquid medication. By slidably pulling the plunger 2, the liquid medication is withdrawn from the bottle to the barrel 1 through the medicament-dispensing needle unit 3. The medicament-dispensing needle unit 3 is detached from the barrel 1 and the injection needle unit 4 is then coupled with the barrel 1. Accordingly, the safety disposable injector further comprises a locking means for locking the injection needle unit 4 with the barrel 1, wherein after the injection needle unit 4 is coupled to the barrel 1, the injection needle unit 4 cannot be detached from the barrel 1. The locking means comprises an outer ratchet 4c4 outwardly protruded from the outer circumferential surface of the injection needle unit 4, and correspondingly, an inner ratchet 1b protruded from the inner circumferential surface of the barrel 1, wherein when the injection needle unit 4 is rotatably engaged with the barrel 1 at one direction, the outer ratchet 4c4 is engaged with the inner ratchet 1b to prevent the injection needle unit 4 being reversely rotated back to an opposite direction. In other words, the injection needle unit 4 can only coupled with the barrel 1 but cannot detached from the barrel 1 once the injection needle unit 4 is coupled thereto. Once the injection needle unit 4 is coupled with the barrel 1, the plunger 2 is slidably pushed to administer injection through the injection needle unit 4. Accordingly, the plunger 2, having a tubular structure, comprises a plunger body and a tubular plunger stopper 2a coupled at a top end of the plunger body of plunger 2 and adapted to being pushed into the interior of the plunger 2 when a downward pushing force is applied at the plunger stopper 2a. When the plunger 2 is pushed toward the injection needle unit 4, the plunger stopper 2a of the plunger 2 will bias against the bottom end of the injection needle base 4b. When the pushing force is kept applying at the plunger body of the plunger 2, the plunger stopper 2a is forced and gradually pushed back into the interior of the plunger body of the plunger 2. At the mean time, the top end of the plunger body of the plunger 2 is aligned with the sealing element 4f and is slidably inserted into the gap between the bottom end of the base sleeve 4c and the bottom end of the injecting needle base 4b. When the pushing force is kept applying at the plunger body of the plunger 2, the top end of the plunger 2 will push against the sealing element 4f until the sealing element 4f is moved offset to its original position so as to dislocate the sealing element 4f. Therefore, the frictional holding force of the sealing element 4f will be released to free the engagement between the base sleeve 4c and the injecting needle base 4b. It is worth mentioning that all the liquid medication will be discharged from the barrel 1 at this position. Once the frictional holding force of the sealing element 4f is released, the compressed compression spring 4d will return back to its original form to apply the spring force against the injecting needle base 4b. Therefore, the injecting needle base 4b, including the injecting needle body 4a, will be pushed into the interior of the plunger body of the plunger 2 through the sealing element 4f so as to retract the injecting needle body 4a into the barrel 1. It is worth mentioning that the injecting needle base 4b will also push the plunger stopper 2a together into the interior of the plunger body the plunger 2. In other words, when the injecting needle body 4a is rapidly retracted into the barrel 1, i.e. also the interior of the plunger 2, via the spring force, the injecting needle body 4a will be pulled back from the patient skin to complete the injection process. It is worth mentioning that the length of the plunger 2 is longer than the length of the injecting needle body 4a such that the entire injecting needle body 4a is retracted into the interior of the plunger 2. Therefore, no protection cap is required for covering the top opening of the barrel 1 to prevent the tip of the used injecting needle body 4a, so as to avoid an injury by accidental needle sticks. In addition, the used injection needle unit 4 cannot be re-assembled back to the barrel 1 to prevent the repeated use of the injection needle unit 4 for ensuring the injection needle unit 4 as a one-time use device.

### Embodiment 2

Embodiment 2 is a modification of the above embodiment, wherein the safety disposable injector further comprises a medicament-dispensing needle protection sleeve 3c for covering the medicament-dispensing needle unit 3. Accordingly, the medicament-dispensing needle protection sleeve 3c is coupled with the medicament-dispensing needle base 3b to enclose the medicament-dispensing needle body 3a within the medicament-dispensing needle protection sleeve 3c. The medicament-dispensing needle base 3b further has a handling wing 3b2, a rhombus cross section (non-circular cross section) 3b4, or knurled outer surface at the outer surface of the medicament-dispensing needle base 3b for enabling the operator to hold and turn the medicament-dispensing needle base 3b to attach to or detach from the barrel 1. Accordingly, the tip of the medicament-dispensing needle body 3a can be formed as a slanted tip or a conical tip 3a1 with a side aperture 3a2 at the circumferential surface of the tip portion.

### Embodiment 3

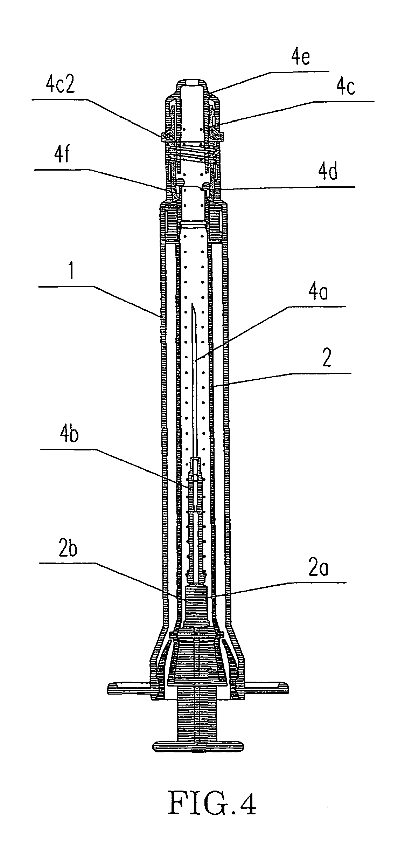

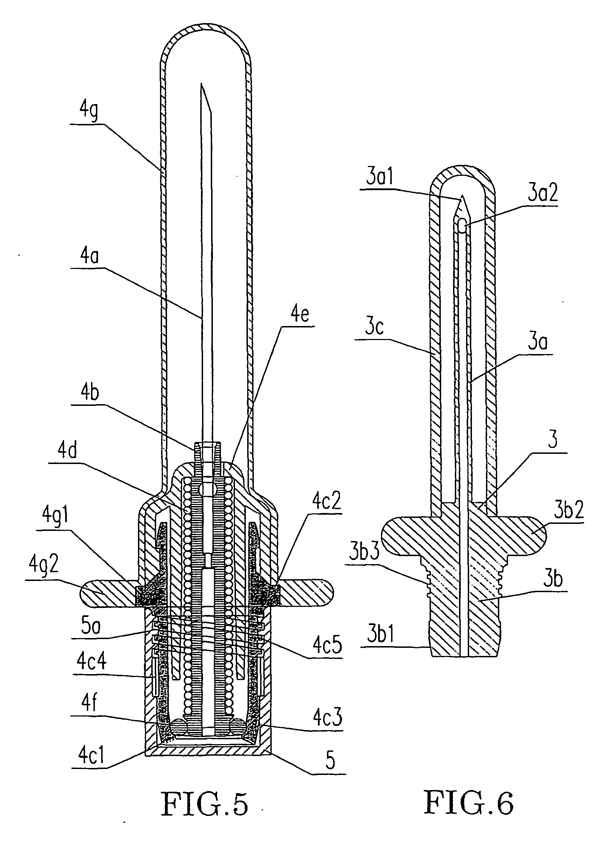

Embodiment 3 is another modification of the above embodiment, as shown in Figs. 4 and 5, wherein the safety disposable injector further comprises an injecting needle protection sleeve 4g for covering the injecting needle unit 4. Accordingly, the injecting needle protection sleeve 4g is coupled with the base sleeve 4c to enclose the injecting needle body 4a within the injecting needle protection sleeve 4g. The injecting needle unit 4 further comprises an inner tongue-groove structure 4g1 provided at an inner surface of the injecting needle protection sleeve 4g and an outer tongue-groove structure 4c2 provided at an outer surface of the base sleeve 4c. Therefore, when the injecting needle protection sleeve 4g is coupled with the base sleeve 4c to enclose the injecting needle body 4a, the inner tongue-groove structure 4g1 is interlocked with the outer tongue-groove structure 4c2. The injecting needle protection sleeve 4g further has a handling wing 4g2, a rhombus cross section (non-circular cross section) 4g3, or knurled outer surface for enabling the operator to hold and turn the injection needle unit 4 to attach to the barrel 1. It is worth mentioning that before the injection needle unit 4 is attached to the barrel 1, the base sleeve 4c is covered and protected by a base cover 5. The base cover 5 can be removed from the base sleeve 4c in order for the injection needle unit 4 attaching to the barrel 1. Preferably, the base cover 5 has an inner threaded portion 5a rotatably and detachably coupled with the base sleeve 4c.

### Embodiment 4

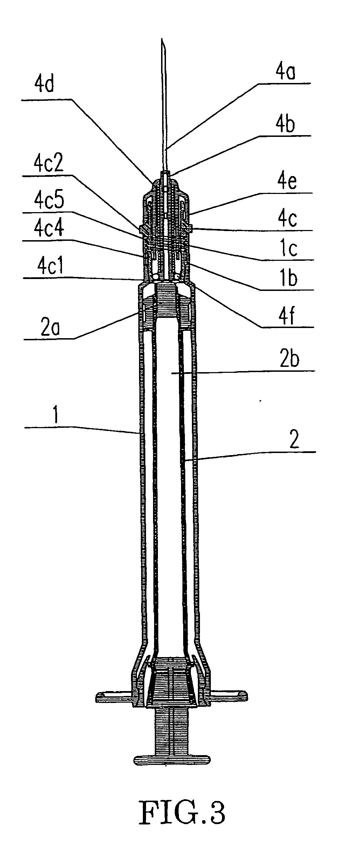

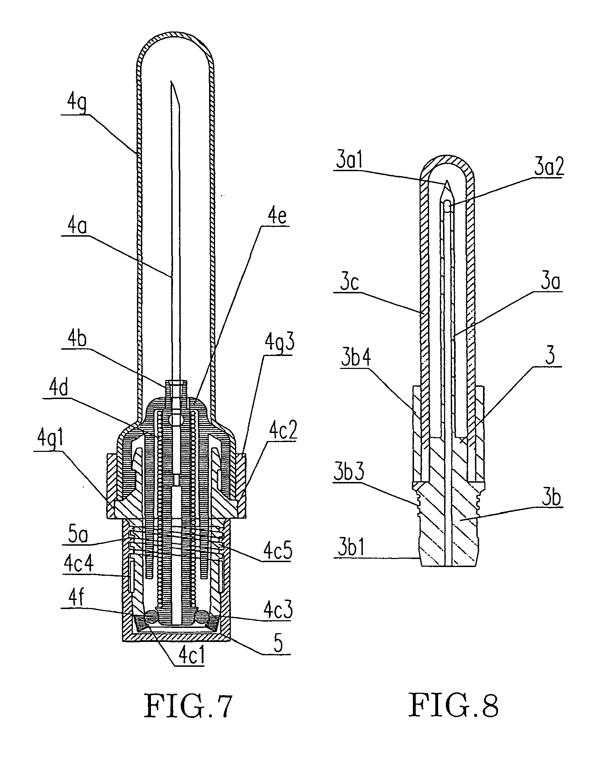

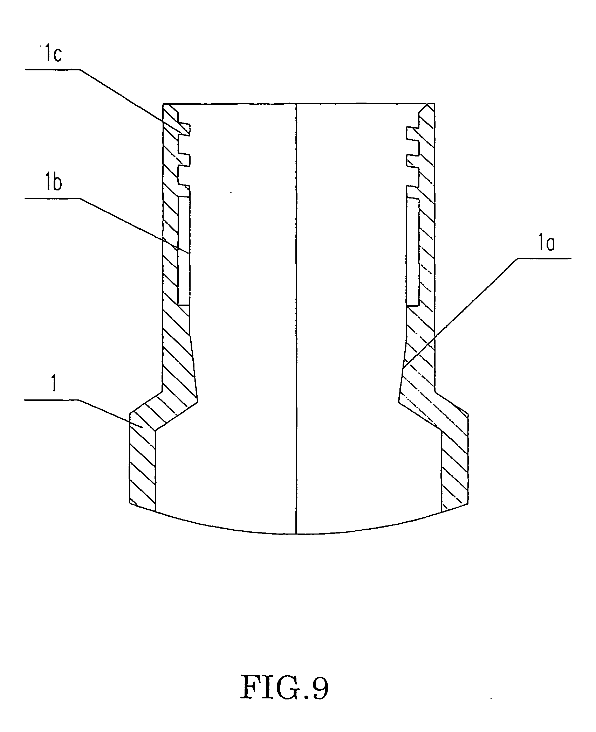

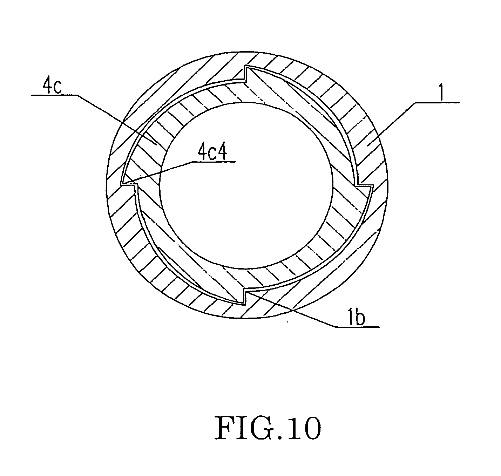

Embodiment 4 is another modification of the above first and third embodiments, as shown in Figs. 3, 4, 7 and 9, wherein the automatic retraction mechanism of the injecting needle unit 4 further comprises an inner ratchet 1b provided at the inner surface of the barrel 1 and an outer ratchet 4c4 provided at the outer surface of the base sleeve 4c. When the base sleeve 4c is rotatably coupled with the barrel 1, the inner ratchet 1b is engaged with the outer ratchet 4c4. Therefore, the base sleeve 4c can only rotate at one direction to couple with the barrel 1, as shown in Fig. 10, so as to prevent the base sleeve 4c being detached from the barrel 1 by rotating the base sleeve 4c at the reverse direction. In other words, the present invention prevents the injecting needle unit 4 being re-used by disassembling the injecting needle unit 4 from the barrel 1 after use. Therefore, the present invention ensures the injecting needle unit 4 being used for one single time only.

One skilled in the art will understand that the embodiment of the present invention as shown in the drawings and described above is exemplary only and not intended to be limiting.

It will thus be seen that the objects of the present invention have been fully and effectively accomplished. It embodiments have been shown and described for the purposes of illustrating the functional and structural principles of the present invention and is subject to change without departure from such principles. Therefore, this invention includes all modifications encompassed within the scope of the following claims.

## Claims

1. A safety disposable injector, comprising:
a barrel 1 having a chamber,
a plunger 2 slidably coupled within said chamber of said barrel 1 in a reciprocal manner, wherein said plunger 2 has a tubular structure and comprises a plunger stopper 2a provided at a top end of said plunger 2;
a medicament-dispensing needle unit 3 comprising a medicament-dispensing needle body 3a and a medicament-dispensing needle base 3b, wherein said medicament-dispensing needle body 3a is extended from said medicament-dispensing needle base 3b;
and an injecting needle unit 4, wherein said medicament-dispensing needle unit 3 and said injecting needle unit 4 are selectively coupled with said barrel 1 in sealed manner;
wherein said injecting needle unit 4 comprises an injecting needle body 4a, an injecting needle base 4b, a base sleeve 4c, a sealing element 4f, a compression spring 4d, and a spring seat 4e, wherein said base sleeve 4c, which is a tubular member, comprises a retention protrusion 4c1 protruded from an inner wall of said base sleeve 4c at a bottom end thereof, wherein said sealing element 4f is supported within said base sleeve 4c by said retention protrusion 4c1 so as to retain said sealing element 4f at the bottom end of the base sleeve 4c, wherein said injecting needle base 4b is held within an interior cavity of said base sleeve 4c, wherein said spring seat 4e is coupled at a top end of said base sleeve 4c, wherein said sealing element 4f is sealed and retained between said bottom end of said base sleeve 4c and said bottom end of said injecting needle base 4b to provide a sealing effect therebetween and to apply a frictional holding force against said injecting needle base 4b to withstand a compression force of said compression spring 4d.
wherein said safety disposable injector further comprises a sealer element provided at said barrel 1 to seal with said medicament-dispensing needle unit 3 and said injecting needle unit 4 when one of said medicament-dispensing needle unit 3 and said injecting needle unit 4 is coupled with said barrel 1.
wherein a top opening end portion of said barrel 1 has an inner diameter gradually reducing toward said bottom end thereof to form an inner conical opening 1a at an inner surface of said barrel 1, wherein, correspondingly, each of said medicament-dispensing needle unit 3 and said injecting needle unit 4 further has an outer conical platform 3b1, 4c3 formed at an outer circumferential surface of said bottom end of each of said medicament-dispensing needle unit 3 and said injecting needle unit 4 to seal and engage with said inner conical opening 1a of said barrel 1.
wherein said barrel 1 has an inner conical opening 1a, and correspondingly, each of said medicament-dispensing needle unit 3 and said injecting needle unit 4 further has an outer conical platform 3b1, 4c3, wherein a taper of said inner conical opening 1a of said barrel 1 is larger than a taper of each of said outer conical platform 3b1, 4c3 of said medicament-dispensing needle unit 3 and said injecting needle unit 4.
wherein said medicament-dispensing needle base 3b further comprises a means for enabling an operator to hold and turn said medicament-dispensing needle base 3b to attach to or detach from the barrel 1, wherein said means comprises a structure selected from the group consisting of a handling wing 3b2, a rhombus cross section 3b4, and knurled outer surface.
wherein said injecting needle unit 4 further comprises an injecting needle protection sleeve 4g for covering said injecting needle unit 4, wherein said injecting needle protection sleeve 4g is coupled with said base sleeve 4c to enclose said injecting needle body 4a within said injecting needle protection sleeve 4g, wherein said injecting needle unit 4 further comprises an inner tongue-groove structure 4g1 provided at an inner surface of said injecting needle protection sleeve 4g and an outer tongue-groove structure 4c2 provided at an outer surface of said base sleeve 4c to engage with said inner tongue-groove structure 4g1.

2. The safety disposable injector, as recited in claim 1, wherein said injecting needle protection sleeve 4g further comprises a means for enabling an operator to hold and turn said injection needle unit 4 to attach to said barrel 1, wherein said means comprises a structure selected from the group consisting of a handling wing 4g2, a rhombus cross section 4g3, and knurled outer surface.

## Patentansprüche

1. Ein Sicherheitseinwegs-Injektor, umfassend:
einen Zylinder 1 mit einer Kammer,
einen Kolben 2 verschiebbar in der Kammer des Zylinders 1 in einer reziproken Art und Weise gekoppelt, wobei der Kolben 2 eine rohrförmige Struktur umfasst und aus einem Stopfen 2a an einem oberen Ende des Kolbens 2 besteht;
ein Arzneimittel-Abgabenadeleinheit 3 mit einem Medikamentenabgabenadelkörper 3a und einer Medikamentenabgabenadelbasis 3b, wobei das Arzneimittel-Abgabenadelkörper 3a erstreckt sich von der Medikamentenabgabenadelbasis 3b;
und eine Injektionsnadeleinheit 4, wobei das Arzneimittel-Abgabenadeleinheit 3 und der Injektionsnadel Einheit 4 selektiv in Verbindung mit dem Zylinder 1 in dichter Weise sind;
wobei die Injektionsnadel Einheit 4 umfasst einen Injektionsnadelkörper 4a, eine Injektionsnadel Basis 4b, 4c eine Sockelhülse ein Dichtelement 4f, eine Druckfeder 4d, und einen Federsitz 4e, wobei die Basishülse 4c, die ein rohrförmiges ist Element umfasst einen Rückhaltevorsprung 4c1 an einem unteren Ende von einer Innenwand der Sockelhülse 4c vorsteht davon, wobei das Dichtelement 4f getragen innerhalb der Basishülse 4c durch die Rückhaltevorsprung 4c1, um das Dichtungselement 4f und .......am unteren Ende der Basishülse 4c, wobei die Injektionsnadel Basis 4b ist in einem inneren Hohlraum des Sockelhülse 4c, wobei der Federsitz 4e ist an einem oberen Ende der Basis verbunden gehaltenen Hülse 4c, wobei das Dichtungselement abgedichtet ist 4f und zwischen dem unteren Ende der Basishülse 4c unteren Ende der Injektionsnadel Basis 4b, eine Dichtungswirkung dazwischen bereitzustellen und um eine Reibungshaltekraft gegen die Injektionsnadelgrund 4b, um eine Kompressionskraft der Druckfeder 4d standgehalten gelten;
wobei der Sicherheitseinweg-Injektor weiter ein Dichtungselement umfasst das Rohr 1 mit Abdichtung der Medikamentenabgabenadeleinheit 3 und der Injektionsnadel Einheit 4 umfasst, wenn einer der Arzneimittel-Abgabenadeleinheit 3 und die Einspritz Nadeleinheit 4 mit dem Zylinder 1 verbunden ist;
wobei eine obere Öffnung Endabschnitt des Zylinders 1 einen Innendurchmesser allmählich verringert zu dem unteren Ende davon an einem inneren konischen Öffnung 1a an einer Innenfläche der Form der Trommel 1, wobei, Entsprechend jeder der Arzneimittel-Abgabenadeleinheit 3 und der Injektionsnadel Einheit 4 weiter eine äußere konische Plattform 3b1, 4c3 an einer Außenumfangsfläche ausgebildet ist, am unteren Ende von jedem der Arzneimittel-Abgabenadeleinheit 3 und der Injektionsnadel Einheit 4 zu versiegeln und sich mit der inneren konischen Öffnung 1a des Zylinders 1;
wobei der Zylinder 1 hat eine innere konische Öffnung 1a und entsprechend jeder der Arzneimittel-Abgabenadeleinheit 3 und der Injektionsnadel Einheit 4 hat ferner einen äußeren konischen Plattform 3b1, 4c3, wobei eine Verjüngung der inneren konischen Öffnung 1a des Zylinders 1 ist größer als eine Verjüngung von jedem der äußeren konischen Plattform 3b1, 4c3 des Medikamentenabgabenadeleinheit 3 und der Injektionsnadel Einheit 4;
wobei das Arzneimittel-Abgabenadelbasis 3b ferner eine Einrichtung zum Ermöglichen einer Bedienungsperson zu halten und wiederum das Arzneimittel-Abgabenadelbasis 3b zu befestigen oder lösen sich von der Trommel 1, wobei die folgendes umfaßt eine Struktur, ausgewählt aus der Gruppe, bestehend aus einem Handhabungsflügel 3b2, einer Raute Querschnitt 3b4 und gerändelte Außenfläche aufweist;
wobei die Injektionsnadel Einheit 4 umfasst ferner ein Einspritzen von Injektions- Nadelschutzhülse 4g zum Abdecken der Injektionsnadel Einheit 4, bei der die Injektionsnadel Schutzhülse 4g ist mit der Basishülse 4c an den umschließen gekoppelten Injektionsnadel Körper 4a in die Injektionsnadelschutzhülse 4g, wobei die Injektionsnadel Einheit 4 ferner eine innere Nut-Feder-Struktur 4g1 an einer Innenfläche des genannten Injektionsnadel Schutzhülse 4g und eine äußere Nut-Feder-Struktur 4c2 auf eine vorgesehen ist Außenfläche der Trägerhülse 4c zum Eingriff mit dem inneren Nut-Feder-Struktur 4g1.

2. Der Sicherheitseinwegs-Injektor nach Anspruch 1 , wobei die Injektionsnadel Schutzhülse 4g weiter eine Einrichtung zum Ermöglichen einer Bedienungsperson zu halten und wiederum die Injektionsnadel Einheit 4 um sie an dem Zylinder 1 befestigen, wobei die Einrichtung eine Struktur umfasst, ausgewählt aus der Gruppe, bestehend aus einem Flügel Handhabung 4g2, eine Raute Querschnitt 4g3, und gerändelte Außenfläche.

## Revendications

1. Un injecteur de sécurité jetable, comprenant:
un cylindre 1 possédant une chambre;
un piston 2 couplé de manière réciproquement coulissante à l'intérieur de ladite chambre dudit cylindre 1, ledit piston 2 possédant une structure tubulaire et comprenant un fouloir de piston 2a fourni au niveau d'une extrémité supérieure dudit piston 2;
une unité d'aiguille distributrice de médicament 3 comprenant un corps d'aiguille distributrice de médicament 3a et une base d'aiguille distributrice de médicament 3b, ledit corps d'aiguille distributrice de médicament 3a s'étendant depuis ladite base d'aiguille distributrice de médicament 3b;
et une unité d'aiguille à injection 4, ladite unité d'aiguille distributrice de médicament 3 et ladite unité d'aiguille à injection 4 étant sélectivement couplées avec ledit cylindre 1 de manière hermétique;
dans lequel ladite unité d'aiguille à injection 4 comprend un corps d'aiguille à injection 4a, une base d'aiguille à injection 4b, une chemise de base 4c, un élément obturant 4f, un ressort de compression 4d et une cuvette de ressort 4e, dans lequel ladite chemise de base 4c, qui est un membre tubulaire, comprend une projection de rétention 4c1 en projection depuis une paroi interne de ladite chemise de base 4c au niveau de son extrémité inférieure, dans lequel ledit élément obturant 4f est soutenu à l'intérieur de ladite chemise de base 4c par ladite projection de rétention 4c1 de sorte à retenir ledit élément obturant au niveau de l'extrémité inférieure de la chemise de base 4c, dans lequel ladite base d'aiguille à injection 4b est maintenue à l'intérieur d'une cavité interne de ladite chemise de base 4c, dans lequel ladite cuvette de ressort 4e est couplée au niveau d'une extrémité supérieure de ladite chemise de base 4c, dans lequel ledit élément obturant 4f est étanchéifié et maintenu entre ladite extrémité inférieure de ladite chemise de base 4c et ladite extrémité inférieure de ladite base d'aiguille à injection 4b pour fournir un effet obturateur entre elles et appliquer une force de retenue frictionnelle contre la base d'aiguille à injection 4b pour supporter une force de compression exercée par ledit ressort de compression 4d;
dans lequel ledit injecteur de sécurité jetable comprend en outre un élément obturant fourni au niveau dudit cylindre 1 pour étanchéification avec ladite unité d'aiguille distributrice de médicament 3 et ladite unité d'aiguille à injection 4 lorsque l'une parmi ladite unité d'aiguille distributrice de médicament 3 et ladite unité d'aiguille à injection 4 est couplée avec ledit cylindre 14;
dans lequel une portion d'extrémité supérieure formant ouverture dudit cylindre 1 possède un diamètre interne se réduisant graduellement vers son extrémité inférieure pour former une ouverture conique interne 1a au niveau d'une surface interne dudit cylindre 1, dans lequel, en correspondance, chacune parmi ladite unité d'aiguille distributrice de médicament 3 et ladite unité d'aiguille à injection 4 possède en outre une plateforme conique externe 3b1, 4c3 formée au niveau d'une surface circonférentielle externe de ladite extrémité inférieure de chacune desdites unité d'aiguille distributrice de médicament 3 et unité d'aiguille à injection 4 pour étanchéifier et s'engager avec ladite ouverture conique interne 1a dudit cylindre 1;
dans lequel ledit cylindre 1 possède une ouverture conique interne 1a et, en correspondance, chacune parmi ladite unité d'aiguille distributrice de médicament 3 et ladite unité d'aiguille à injection 4 possède en outre une plateforme conique externe 3b1, 4c3, dans lequel une conicité de ladite ouverture conique interne 1a dudit cylindre 1 est supérieure à une conicité de chacune desdites plateformes coniques externes 3b1, 4c3 desdites unité d'aiguille distributrice de médicament 3 et unité d'aiguille à injection 4;
dans lequel ladite base d'aiguille distributrice de médicament 3b comprend en outre un moyen pour permettre à un usager de tenir et tourner ladite base d'aiguille distributrice de médicament 3b pour l'attacher au, ou la détacher du, cylindre 1, ledit moyen comprenant une structure sélectionnée parmi le group consistant d'une ailette de manutention 3b2, une section de coupe en losange 3b4 et une surface externe moletée;
dans lequel ladite unité d'aiguille à injection 4 comprend en outre une chemise de protection d'aiguille à injection 4g pour couvrir ladite unité d'aiguille à injection 4, ladite chemise de protection d'aiguille à injection 4g étant couplée avec ladite chemise de base 4c pour enfermer ledit corps d'aiguille à injection 4a à l'intérieur de ladite chemise de protection d'aiguille à injection 4g, ladite unité d'aiguille à injection 4 comprenant en outre une structure interne de languette et rainure 4g1 fournie au niveau d'une surface interne de ladite chemise de protection d'aiguille à injection 4g et une structure externe de languette et rainure 4c2 fournie au niveau d'une surface externe de ladite chemise de base 4c de sorte à s'engager avec ladite structure interne de languette et rainure 4g1.

2. Le kit de montage selon la revendication 1, dans lequel ladite chemise de protection d'aiguille à injection 4g comprend en outre un moyen pour permettre à un usager de tenir et tourner ladite unité d'aiguille à injection 4 pour l'attacher au cylindre 1, ledit moyen comprenant une structure sélectionnée parmi le group consistant d'une ailette de manutention 4gb2, une section de coupe en losange 4g3 et une surface externe moletée.
